# EUROPEAN PATENT SPECIFICATION

(11) **EP 2 318 014 B1**
(45) Date of publication and mention of the grant of the patent: **14.09.2016**
(21) Application number: 09787621.3
(22) Date of filing: 20.07.2009
(51) Int. Cl.: A61K 31/7008, A61K 31/722, A61P 19/02

(54) **NEW COMPOSITION FOR TREATING AUTOIMMUNE DISORDERS**
NEUE CHITO-OLIGOMER ZUBEREITUNG ZUR MEDIZINISCHEN VERWENDUNG
NOUVELLE COMPOSITION POUR LE TRAITEMENT DE TROUBLES AUTO-IMMUNS

(30) Priority: 18.07.2008 IS 8751
(43) Date of publication of application: 11.05.2011
(73) Proprietor: Genís hf., 580 Siglufjördur (IS)
(72) Inventor: GISLASON, Johannes, IS-105 Reykjavik (IS); EINARSSON, Jon, M., IS-108 Reykjavik (IS); NG, Chuen How, IS-108 Reykjavik (IS)
(74) Representative: Arnason Faktor
(86) International application number: PCT/IS2009/000009
(87) International publication number: WO 2010/007631

(56) References cited:
- WO-A-2005/034961
- WO-A-2006/134614
- WO-A1-03/026677
- CHEN ET AL: "Updated Therapy in Elderly Patients with Knee Osteoarthritis" INTERNATIONAL JOURNAL OF GERONTOLOGY,, vol. 1, no. 1, 1 March 2007 (2007-03-01), pages 31-39, XP025469976 ISSN: 1873-9598 [retrieved on 2007-03-01]
- MATHESON ANNA J ET AL: "Glucosamine: A Review of its Use in the Management of Osteoarthritis" DRUGS AND AGING, ADIS INTERNATIONAL LTD, NZ, vol. 20, no. 14, 1 January 2003 (2003-01-01), pages 1041-1060, XP009096907 ISSN: 1170-229X
- SHIKHMAN A R ET AL: "Chondroprotective activity of N-acetylglucosamine in rabbits with experimental osteoarthritis" ANNALS OF THE RHEUMATIC DISEASES, vol. 64, no. 1, January 2005 (2005-01), pages 89-94, XP002544557 ISSN: 0003-4967

## Description

### Field of the invention

The present invention relates to a new chitooligomer composition for medical use. More specifically the invention relates to biomaterials and medicaments made from chitin which synergistically promote regenerative and anti-inflammatory effects *in vivo* and *in vitro.*

### Technical background and prior art

The development of first generation of biomaterials, although having acceptable mechanical strength, resulted in passive contribution to tissue healing, as their function was confined to support tissue replacement. These first generation of biomaterials released degradative debris which, in turn, led to the onset of chronic inflammation, pain, and effusion. The main purpose of the development of second generation of biodegradable biomaterials was to eliminate the chronic, long-term problems that resulted from the continuous release of debris particles originating in the first generation materials. This resulted in implantable biodegradable materials offering tissue-supporting properties for the desired length of time (months), and thereafter they underwent local degradation by the surrounding tissues and were subsequently excreted outside the body via urine and/or feces. An example of this technology is biodegradable vascular sutures in use today.

Despite the good biocompatibility properties of the second generation of biomaterials, their contribution to the promotion of tissue healing is limited, due to their passive role in the biologic processes involved in tissue healing and regeneration. The need of developing a more modern set of a third generation of biomaterials resides in the desire to develop compounds, which will actively contribute to the process of tissue healing and regeneration.

Applicant's prior application WO 2006/134614 discloses a method to make highly purified partially deacetylated chitin biomaterial for therapeutic applications. The oligomeric compositions produced by this method are herein referred to as "Therapeutic Chitooligosaccharides" (T-ChOS). The therapeutic chitooligosaccharides are characterized by their chain length of 4-20 monomer residues and their specific sequence of D-glucosamine (D) and *N*-acetyl-D-glucosamine (A), where the internal part of the oligomer having at least enough glucosamine (D) residues to avoid that an N-acetyl glucosamine residue (A) is adjacent to another N-acetyl glucosamine residue (such as AA).

There are basically two ways for a damaged or diseased tissue to repair or heal. One is through fibrosis leading to scar formation. Since the fibroblasts involved in the formation of fibrotic tissue do not possess the functions of the original tissue cells, this repair pathway leads to impaired tissue function. Inflammatory cytokines are usually involved in fibrosis.

YKL-40 is thought to play a role in fibrosis through chemotaxis and stimulation of fibroblast growth.

The alternative pathway of tissue repair is tissue regeneration or functional tissue repair. In this pathway, functional cells of the particular tissue are regenerated, probably from tissue specific progenitor cells, resulting in regeneration of a healthy functional tissue.

In bone tissue, osteogenic and osteoinductive biomaterials or devices promote bone growth by inducing proliferation of progenitor cells capable of developing into cartilage and/or bone tissue and are characterized as materials that not only fill voids but also exhibit intrinsic properties that cause or induce the body to produce new bone within the scaffold provided by the biomaterial composition. These materials not only act as a scaffold, but also contain proteins or other substances that induce the formation of new bone. Auto grafts have been used in orthopedic surgical procedures for many years, and are the most common method of assisting the body's regenerative ability. Only one commercially available product demonstrates true osteoinductive properties, the recently introduced device from Medtronic called InFUSE^{®} incorporating a recombinant version of human bone morphogenetic protein (BMP). Furthermore, Glucosamine has been extensively used for treating osteoarthritis.

Agents capable of promoting tissue regeneration through systemic administration (such as orally, subcutaneously, intramuscularly, intravenously etc.) have until now not been discovered.

### Summary of the invention

In an earlier patent application (WO 2006/057011) the inventors have demonstrated that partially deacetylated chitin derivatives, implanted into a bone defect, are capable of inducing endochondral ossification. The present invention demonstrates regenerative effect of T-ChOS and T-ChOS + glucosamine in *in vitro* and *in vivo* models demonstrating effect of the specific aminosugars through systemic administration. Additionally it is demonstrated that dimers and trimers of glucosamine and N-acetyl glucosamine (DP2 and DP3) exhibit an inhibitory effect. The present invention also discloses a surprising synergistic effect of T-ChOS and glucosamine as a bioactive composition facilitating anti-inflammation and tissue regeneration in rheumatic arthritis (RA). It is further demonstrated in the examples that the combination of T-ChOS and glucosamine constitutes an active agent for promoting and enhancing collagen type II formation in cartilage explants, apparently through interaction with YKL-40, a member of the CLP protein family. It is an object of the present invention to provide a composition, use and a method for reducing inflammation and promoting tissue repair or tissue regeneration through a synergistic effect of T-ChOS and glucosamine and/or N-acetyl glucosamine systemically or locally administered in humans and other mammals in need thereof.

In a first aspect of the invention a composition is provided for treatment of rheumatic arthritis, the composition comprises therapeutically active chitooligomers (T-ChOS) of N-acetyl glucosamine (A) and glucosamine (D), where the chitooligomers comprise hetero-chitooligomers which have to fulfill the following criteria: the oligomers have a chain length in the range of 5-20 monomer residues and each oligomer chain can have two N-acetyl glucosamine residues (AA) on either or both ends of the oligomer chain. The sequence of the internal chain (the portion within the terminal two residues on each end) is such that an N-acetyl glucosamine residue (A) is not adjacent to another N-acetyl glucosamine residue (such as AA). The composition further comprises glucosamine monomers, wherein the ratio between monomers and oligomers is in the range between about 1:5 to about 5:1, and wherein the degree of deacetylation (DD) of said therapeutically active chitooligomers is in the range between 30-60%. The composition according to the first aspect of the present invention is a biomaterial exhibiting the major criteria of biomaterials, namely biocompatibility, biodegradability, and bioactivity.

The suitable ratio of the therapeutically active chitooligomers (T-ChOS) and the monomer may depend on the specific indication, application, device and dosage form. Broadly, the ratio (weight/weight) between the monomers and oligomers can be in the range of about 1:5 to about 5:1, including the range of about 1:1 to about 5:1. In certain embodiments, the weight amount of monomer is higher and the range may lie in the range between about 1:1 to about 5:1, including the range of about 1:1 to about 4:1, such as the ratio of about 2:1, about 4:1 and more preferably about 3:1. In other embodiments, more is used of the T-ChOS oligomers, such that the ratio of monomer to oligomer is in the range of about 1:1 to about 1:4, including the ratio of about 1:1, about 1:2, and about 1:3.

The present invention also discloses a pharmaceutical composition comprising therapeutically active chitooligomers of N-acetyl glucosamine (A) and glucosamine (D) (T-ChOS) in synergy with added glucosamine and/or N-acetyl glucosamine monomers. The chitooligomer component comprises hetero-chitooligomers as defined above, which have a significant chain length distribution in the range of 4-20 monomer residues and each oligomer chain can have two N-acetyl glucosamine residues (AA) on either or both ends of the oligomer chain. The sequence of the internal chain is such that an N-acetyl glucosamine residue (A) is not adjacent to another N-acetyl glucosamine residue (such as AA). The composition further comprises glucosamine monomers and/or N-acetyl glucosamine, preferably in the weigh ratio as described above. This pharmaceutical composition can be in the form of a powder, a suspension, a gel, a sol, aerosol, a paste, a film, foam, a pill, and a capsule. The pharmaceutical composition can further comprise a pharmaceutically acceptable excipient.

The present invention also discloses use of the compositions of the invention for the manufacture of a biomaterial/medicament for anti-inflammation and tissue regeneration.

The present invention also discloses a use of the compositions for the manufacture of a biomaterial/medicament for regulating collagen synthesis in order to prevent scar formation in tissue repair. The composition comprises therapeutically active chitooligomers of N-acetyl glucosamine (A) and glucosamine (D) (T-ChOS), according to the first aspect of the present invention.

### Description of the present invention

The composition of the present invention is a combination of two chitin materials which show synergistic effect in tissue regeneration. It has been demonstrated that therapeutically active chitooligomers (T-ChOS) bind Chitinase-Like Proteins (CLP) in the body and our data show that CLPs can play a role in the synthesis of collagen in the body and as a result of interaction between CLPs and T-ChOS, collagen synthesis is up-regulated. The collagen Type II synthesis in articular cartilage is initiated by the formation of procollagen, which is synthesized in two alternatively spliced forms PIIANP and PIIBNP, where PIIANP immobilizes TGF-β1, BMP-2 and BMP-4, of the TGF-β super family. This potentially regulates growth factor signaling during development and is a key element for regulation of endochondral bone formation, the natural pathway of bone formation (Endochondral ossification) involving cartilage tissue formation, vascularization of cartilage tissue, enhancing mineralization and subsequent ossification of the induced cartilage tissue forming lamellar bone. Bone Morphogenic Proteins (BMPs) are unwanted during the formation of articular collagen and the first stages of bone formation but are specifically released when the tissue is guided into the pathway of endochondral bone formation.

Since the present invention shows that the presence of DP2-3 blocks the desired biological action of the composition, the T-ChOS component of the composition of the present invention is further characterized in that the amount of monomers, dimers and trimers (DP1, DP2 and DP3) is greatly reduced.

The oligomeric compositions of the present invention represent optimization of the therapeutic activity of the oligomeric compositions including; bioavailability, biostability, and bioactivity. The oligomeric compositions herein referred to as "Therapeutic Chitooligosaccharides" (T-ChOS) have been produced by a method disclosed in WO 2006/134614 to recover highly pure and fully soluble partially deacetylated chitin polymer. A partially deacetylated chitin polymer composition is treated with family 18 endo-chitinase and subsequently filtered to give the desired composition of chain length in the range of 4-20 monomer residues and each oligomer chain can have two N-acetyl glucosamine residues (AA) on either or both ends of the oligomer chain. The sequence of said internal chain is such that an N-acetyl glucosamine residue (A) is not adjacent to another N-acetyl glucosamine residue (i.e. no "AA" pairs are formed in the internal portion of the chain with the two-residue terminal ends which can be AA). As a result of this construction the composition of chain length in the range of 4-20 monomer residues cannot be cleaved by chitinases in the body.

The degree of deacetylation of the oligomers is preferably within the range of about 30-60%, such as about 30%, about 40%, about 50% or about 60%.

The sequential pattern of the therapeutic chitooligomers directly affects their *biological activity*, i.e. how they are transported over biological membranes (bioavailability), how rapidly they break down in living systems (biostability), and how they interact with chitinase like proteins and other specific receptors binding chitinous sequences (bioactivity).

*Bioavailability*, or the ability for a given substance to pass through biological membranes, is related to the hydrophobicity of the molecules. Since all biological membranes are predominantly of a hydrophobic nature, the general rule applies that the more hydrophobic a substance is the better it can penetrate such biological membranes. N-acetyl-glucosamine and fully acetylated chitin oligomers are more hydrophobic than the corresponding glucosamine monomer or highly deacetylated chitosan oligomers, suggesting that chitinous hetero oligomers will possess increased bioavailability with increased acetylation. Hence, the T-ChOS formulations have been optimized to contain a maximum amount of N-acetyl-glucosamine in their molecular structure in order to maximize their bioavailability, without jeopardizing their biostability.

*Biostability* of an organic compound refers to its susceptibility to endogenous enzymes in a living organism and its half-life (t½) in that organism. The more susceptible the less biostable is the compound. In humans, chitinolytic enzymes can be divided into two groups; enzymes with high level chitinolytic specificity like Family 18 chitinases (AMCase, Chitotriosidase), possessing high specific activity, or enzymes with less chitinolytic specificity such as lysozyme and probably some proteases which happen to degrade chitin and chitosan but at lower specific activity. By partial deacetylation, the T-ChOS compositions have been optimized for maximum stability towards hydrolysis by Family 18 chitinase. Since these enzymes require a sequence of two or more consecutive N-acetyl-glucosamine residues as recognition for cleavage, the T-ChOS compositions are specifically optimized to exclude such sequences in the internal part of the molecule.

*Bioactivity* of an organic substance or a ligand is directly linked to the affinity of the ligand to the target receptor triggering the biological response. Little is still known about the biological role of chitinous compounds in the human body, although there are indications that chitin oligomers play a vital role in embryonic development. This suggests that the human genome is capable of expressing specific receptors which are specifically activated when binding to chitin oligomers. The only known chitin binding proteins in the human body, are the chitinase like proteins (CLPs), genetically belonging to the Family 18 chitinases, although a majority of them have lost their enzymatic activity, but still preserving their chitin binding domain.

The term "endochondral ossification" refers to a bone forming process, whereby cartilage develops first yielding the framework of the final bone. The cartilaginous tissue needs less local oxygen tension for its development and maintenance than mature bone tissue and therefore, wherever the blood supply system has not attained its final stage of development, cartilage will supersede bone. Cartilage will only be replaced by new bone after vascularization has reached its advanced stage, guaranteeing essential supply of oxygen to the developing tissues. This process of bone formation is also typical during the embryonic stage, particularly in vertebrae, long bones, sternum, etc.

In the present context the term "medical device" generally refers to an instrument apparatus, implement, machine, contrivance, implant, *in vitro* reagent, or other similar or related article, including a component part, or accessory which is intended for use in the diagnosis of disease or other conditions, or in the cure, mitigation, treatment, or prevention of disease, in humans or other animals, or intended to affect the structure or any function of the body of humans or other animals. In the context herein, the term "*biomaterial product*" is used interchangeably with the term "*medical device*".

The pharmaceutical compositions described herein comprise the therapeutic chitooligomers (T-ChOS) and in synergy with glucosamine and/or N-acetyl-glucosamine. They can be administered systemically and bind to endogenous CLPs, many of which have been shown to or implied as playing a role in several diseases and conditions. Among the diseases and conditions that are associated with elevated expression of CLPs are degenerative diseases (e.g. rheumatoid arthritis) and other diseases including osteoarthritis. The T-ChOS compositions of the invention are found to be useful for treating and/or remedying these diseases as well as conditions relating to bone tissue formation and conditions such as bone regeneration after surgical interventions or trauma. However, together with glucosamine this effect is elevated. The suitable ratio of the T-ChOS ologmers and monomers is preferably as described above.

The composition may further comprise a pharmaceutically acceptable excipient such as processing aid or stability agents, diluents, flavorings, nutrients, or colorants or appropriate additional biologically active or non-active ingredients.

The pharmaceutical composition shall preferably be in a form suitable for oral administration, such as a dry form which can be readily dissolved, e.g. in a glass of water. Such forms include dry powder, a suspension, a gel, a film, foam, a sol, aerosol, granular, flake, fibrous and paste forms. However, the composition can also be contained in pills or capsules. The pharmaceutical compositions can further comprise a pharmaceutically acceptable excipient.

Also disclosed are compositions that are used for the manufacture of a biomaterial/medicament for tissue regeneration such as enhancing bone regeneration in the healing of a fractured or severed bone in a mammal. Such medicament enhances e.g. bone formation through endochondral ossification by activation of tissue specific progenitor cells.

The biomaterial can comprise one or more further component selected from the group consisting of calcium phosphates, including hydroxyapatite, calcium sulphate, sodium tripolyphosphate, alginate, collagen, hyaluronic acid and chitosan polymer.

The composition can be in a form suitable for other forms of systemic administration, such as intramuscular, subcutaneous, or intravenous administration. Such suitable forms are solution forms with a pharmaceutically acceptable carrier or excipient according to standard pharmaceutical practice. Said solution forms are sterile, and the pH is suitably adjusted and buffered. For intravenous use, the total concentration of solute should be controlled to render the preparation isotonic.

The T-ChOS alone can be used as a medicament for directing tissue repair of damaged or diseased tissue away from fibrosis which leads to scar formation, but rather to induce tissue regeneration or functional tissue repair, resulting in regeneration of a healthy functional tissue. This regulation of the pathway of tissue repair is done through control of collagen synthesis in the injured or inflamed tissue.
In an embodiment of the present invention T-ChOS + glucosamine are used as a medicament for reduction of weight gain in post-menopausal women.

In the present context the term "medical device" generally refers to an instrument.
The therapeutic chitooligomers of this invention are particularly useful in biomaterials for various purposes. Besides exhibiting all advantageous features of conventional chitosan (biocompatibility, ability to mix with other components to produce suitable mixtures for medical devices, such as mechanical implants, drug delivery devices, etc.), they possess significantly increased solubility and biological or therapeutic activity due to their high affinity to CLPs in the body, as described above.

Formulation of the biomaterials can suitably include other organic and inorganic components such as various biopolymers (alginates and other polysaccharides etc.), collagen, calcium phosphates, including hydroxyapatite, calcium sulfate, sodium tripolyphosphate, sodium dihydrogen phosphate, sodium glycerol phosphate, calcium oxide, calcium hydroxide and various organic or carboxylic acids etc.

### Detailed description of a preferred embodiment

The present invention will now be disclosed on more detail using examples and figures for enablement of the claimed invention.

### Figures

**Figure 1****.** HPLC analysis (TSK-Oligo column; TosoHaas, Japan) of Oligomin and T-ChOS.
**Figure 2****.** The effect of various aminosugars on accumulated chondroitin sulphate release for day 10-23. Oligomin 200 and 400 µg/ml (Ol 200 and Ol 400) as well as 400 µg/ml glucosamine (D400) show significant reduction of chondroitin sulphate breakdown. Mean values and SEM (standard error of measurement); N(group)=24
**Figure 3****.** The effect of T-ChOS on N-terminal propeptide of type II collagen (PIINP) released to the media between day 20 and day 22. No effect except T-ChOS 50, 100, 200 and 400 µg/ml. Means and SEM; N(group)=4.
**Figure 4****.** The effect of T-ChOS + glucosamine (D) on accumulative N-terminal propeptide of type II collagen (PIINP) released to the media between (day 20 and day 24 pooled). Mean values and SEM; N(group)=8.
**Figure 5****.** The effect of T-ChOS + glucosamine (D), glucosamine and IGF-1 on accumulative chondroitin sulphate release from day 17 to day 24. Means and SEM; n group=16. IGF-1 and T-ChOS + D show significant increase of the accumulative chondroitin sulphate release. Based on data shown in Figure 9.
**Figure 6****.** The dose related induction of T-ChOS on N-terminal propeptide of type II collagen (PIINP) level in media at day 18 to day 25 in human OA cartilage explant. Only T-ChOS showed induction. Asterisks indicate significance of T-ChOS 400 µg/ml to 0 µg/ml by t-test (*=p<0.05, **=p<0.01, ***=p<0.001). Mean values and SEM; N(group)=4.
**Figure 7****.** The effect of three aminosugars on YKL-40 expression for the period (N=4; mean values and SEM).
**Figure 8****.** Expression of YKL-40 related to PIINP expression for the control, glucosamine, Oligomin and T-ChOS groups at day 15 - 25. Only the T-ChOS group is showing significantly higher expression of PIINP (N=20).
**Figure 9****.** The effect of glucosamine, T-ChOS and T-ChOS + glucosamine combination on accumulated ancle diameter (Acc AD) from day 9 to day 17. 0: no treatment, D21: 21 mg/kg/rat glucosamine, T: 7.1 mg/kg/rat T-ChOS, T+D14, 21 and 28: T: 7.1 mg/kg/rat T-ChOS + 14, 21, and 28 mg/kg/rat glucosamine respectively.
**Figure 10****.** The effect of glucosamine, T-ChOS (T) and T-ChOS + glucosamine combination (T+D21) on ankle cartilage damage score. Numbers indicate daily doses (mg/kg rat). Asterics indicate significant difference from 0 by t-test.
**Figure 11****.** The effect of glucosamine, T-ChOS (T) and T-ChOS + glucosamine combination (T+D21) on ankle bone resorption score. Numbers indicate daily doses (mg/kg rat). Asterics indicate significant difference from 0 by t-test.
**Figure 12****.** The effect of glucosamine, T-ChOS (T) and T-ChOS + glucosamine combination (T+D21) on ankle pannus score. Numbers indicate daily doses (mg/kg rat). Asterics indicate significant difference from 0 by t-test.
**Figure 13****.** The effect of glucosamine, T-ChOS (T) and T-ChOS + glucosamine combination (T+D21) on ankle total histopathological score. Numbers indicate daily doses (mg/kg rat). Asterics indicate significant difference from 0 by t-test.
**Figure 14****.** The effect of glucosamine (D21), T-ChOS (T) and T-ChOS + glucosamine combination (T+D) on knee pannus formation. Numbers indicate daily doses (mg/kg rat). Asterics indicate significant difference from 0 by t-test.
**Figure 15****.** The effect of glucosamine (D21), T-ChOS (T) and T-ChOS + glucosamine combination (T+D) on knee bone resorption. Numbers indicate daily doses (mg/kg rat). Asterics indicate significant difference from 0 by t-test.
**Figure 16****.** The effect of glucosamine (D21), T-ChOS (T) and T-ChOS + glucosamine combination (T+D) on knee total histopathological score. Numbers indicate daily doses (mg/kg rat). Asterics indicate significant difference from 0 by t-test.
**Figure 17****.** The effect of Oligomin, T-ChOS alone and T-ChOS in combination of N-acetyl glucosamine (A) or glucosamine (D) on pain and inflammation in a rheumatic arthritis (RA) patient. Ratings are from 0 (no relief) to 10 (complete relief). Daily doses were Oligomin: 2200 mg, T-ChOS: 700 mg, N-acetyl glucosamine (A) or glucosamine (D): 1500 mg. Arrows indicate when monomers were added to the T-ChOS.

### EXAMPLES

### Example 1: Regeneratve effects of specific aminosugars in bovine and human cartilage explants under anabolic and catabolic condititions

The aim of these experiments was to evaluate the effect of aminosugars on cartilage formation in articular cartilage under unstimulated conditions. These experiments could identify possible chondroanabolic effects of these aminosugars as potential osteoarthritis drugs. Insulin-like Growth Factor-1 (IGF-1), 100 ng/ml served as a positive anabolic stimulation control of the cartilage explants.

### MATERIAL AND METHODS

All reagents used were of analytical grade. The culture medium comprised Dulbecco's Modified Eagle Medium (D-MEM) containing penicillin and streptomycin (Life Technologies, US). Human recombinant oncostatin M (OSM) was from Sigma Aldrich (UK), whereas human recombinant tumor necrosis factor α (TNF-α) was from R&D Systems, UK.

### AMINOSUGARS: PRODUCTION AND ANALYSIS

Oligomin. Chitooligosaccharides were produced by Genis at a pilot scale (lot G061023). Briefly, partially deacetylated chitin (DDA 45%) was hydrolyzed by chitinase to near completion. The solution was ultrafiltrated (10 kDa) in order to eliminate chitinase and insolubles and spray-dried.

T-ChOS. Chitooligomer compositions with greatly reduced DP1-4 amount were produced from Oligomin by Genis at a pilot scale (lot G051128). The monomer (DP1 or N-acetyl glucosamine) was eliminated and shorter oligomers were reduced by ultrafiltration. The product was spray dried. N-acetyl glucosamine (A) and glucosamine (D) were purchased from YSK, Japan. For analysis of all aminosugars used, HPLC was applied using Beckman Gold system. TSK-oligo column (TosoHaas, Japan) was used, separating the ChOS by molecular weight (DP1, DP2 etc.). The solvent was 5 mM ammonium hydroxide, pH 10.0, flow rate was 0.5 ml/min, optical absorbance was 205 nm, injection volume was 20 µl and aminosugar concentration was 10 mg/ml.

For cartilage explant experiments, aminosugar concentrations tested were as follows. Oligomin and T-ChOS, 50, 100, 200 and 400 µg/ml in the media. N-acetyl glucosamine (A) and glucosamine (D), 200 and 400 µg/ml in the media. A + D combination were 200 µg/ml each in the media.

### CARTILAGE EXPLANTS

### Anabolic conditions

Bovine articular cartilage explants cultures are used as a model for cartilage degenerative diseases that enables experiments in a robust and simple test-model/assay, where the effect of growth factors and drugs on cartilage metabolism can be investigated [ Olsen, A.K., et al., Anabolic and catabolic function of chondrocyte ex vivo is reflected by the metabolic processing of type II collagen. Osteoarthritis and Cartilage, 2007. 15(3): p. 335-342.].

### PREPARATION OF ARTICULAR CARTILAGE EXPLANTS

The knees were opened under semi-sterile conditions in a LAF bench. Cuts of surface articular cartilage were removed in a one-movement-maneuver, removing only the outermost layer. Too deep cuts will contain underlying subchondral bone /chondrosteous material. Uniform explants were harvested from both femur and tibia. Explants from the cartilage situated at the chondyles were avoided. The explants were transferred into a Petridish containing PBS + pen/strep (penincillin/streptomycin).

### PREPARATION AND CULTURING

The explants were individually weighed and transferred into a sterile 96 well plate under semi-sterile conditions in a flow-bench. Prior, the wells were filled with 200 µL PBS + pen/strep, to keep the explants wet after weighing. Then PBS was removed, and medium containing different types and concentration of aminosugars were applied to the wells, 200µL/well, according to the set-up. Four explant replicates were used for each test. The plates were incubated at 37 °C and 5% CO₂ with shaking 50 rpm. The plates were covered in a CO₂ permeable plastic bag to limit/avoid contamination from spores. The conditioning medium was replaced every 2nd - 3rd day, and the supernatant was transferred into a new 96 wells plate and stored at -20°C until end of experiment. Fresh medium different types and concentration of aminosugars were applied to the wells, 200µL/well, according to the setup every 2nd- 3rd day.

At last day of experiment (22-24 days) the supernatants were removed and cell viability was measured by Alamar Blue, to investigate cell number and viability. Also at last day of experiment the cartilage was processed for extractions of proteins from the cartilage in various assays.

Supernatants were measured for type II collagen formation (PIINP), for aggrecanase-mediated aggrecan degradation. ELISA kits from Nordic Bioscience were used.

### CELL VIABILITY AND CELL NUMBER MEASUREMENTS

To ensure the specific and non-toxic action of aminosugars, the metabolic activity of chondrocytes were measured by Alamar Blue, which previously has shown to correlate to cell number and cell viability.

### EVALUATION OF CARTILAGE TURNOVER

*Aggrecanase mediated aggrecan degradation*: Detection of the aggrecan fragment 374ARGS: The ELISA detecting the aggrecanase-derived fragments of the N-terminal 374ARGS combines two monoclonal antibodies in a sandwich ELISA system. The method followed is described by Karsdal et al. Arthritis & Rheumatism, 2007. 56(5): p. 1549-1558

### TOTAL PROTEOGLYCAN CONTENT - MEASUREMENTS OF SGAG

For detection of sulfated glycosaminoglycans (sGAG) that is chondroitin sulphate release, the quantitative dye-binding assay for in vitro analysis of GAG release was used according to the manufacturer's instructions (Wieslab, S).

### COLLAGEN TYPE II FORMATION: PIINP ELISA

Collagen type II formation is measured by a specific ELISA assay based on an antibody that recognizes an epitope on the PIINP molecule outside the exon 2 (PIIANP). Therefore the ELISA is not specific for the IIA or the IIB form but reacts with both forms.

### EXTRACTION OF CARTILAGE:

The cartilage was extracted to determine the cartilage content of various proteins, in comparison of that secreted to the medium.

Cartilage explants were frozen in liquid nitrogen. The frozen explants were pulverized using Bessman Tissue Pulverizer according to the instructions supplied by the manufacturer. The powder was placed in 14 ml tube using frozen scalpel. Ice-cold Digestive Buffer (50mM Tris·HCl buffer, pH 7.4 containing 0.1M NaCl and 0.1% Triton X-100) was added and the solution homogenized for 30 sec using Polytron PT-MR 3000 homogenizer (Brinkmann, Littau-Switzerland).The homogenate was then centrifuged at 15,000 rpm for 20 min and the supernatant collected and stored at -80°C until further analysis.

### TOTAL COLLAGEN CONTENT: MEASUREMENTS OF HYDROXYPROLINE

Total collagen is evaluated by measuring the content of hydroxyproline in the explants using a modified version of the method described by Podenphant. (Podenphant, J., N. Larsen, and C. Christiansen, An easy and reliable method for determination of urinary hydroxyproline.. Clinica Chimica Acta 1984. 142: p. 145-148).

### Results

### Aminosugar analysis.

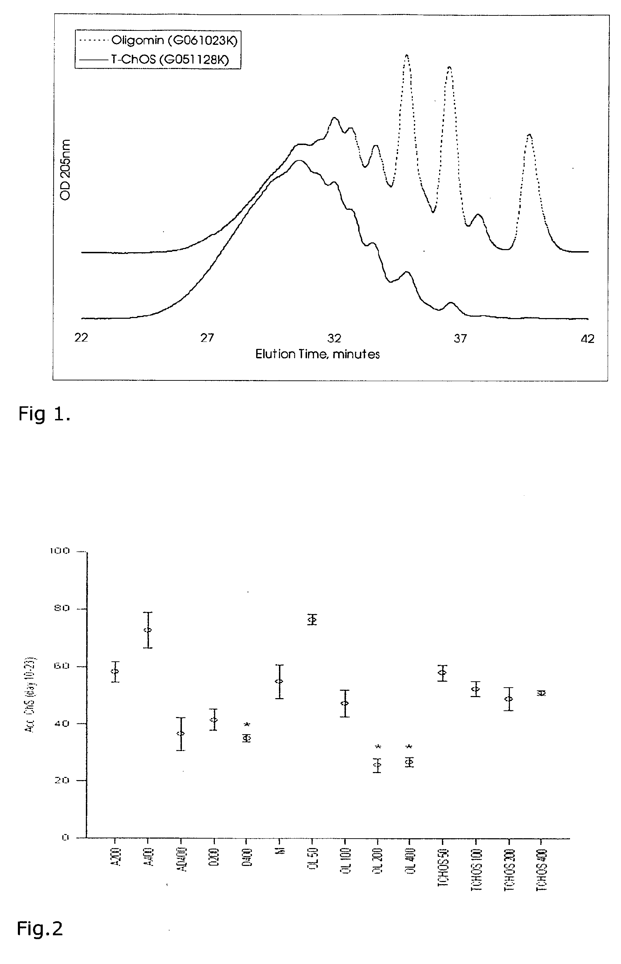

Figure 1 shows the comparison of Oligomin and T-ChOS chitooligosaccharides. For Oligomin monomer to trimer (DP1-DP3) was the main component (58.4%) and DP4 and greater was 41.6%.

For T-ChOS no monomer (DP1) was detected and the DP1-DP3 fraction was only 6.1%. Octamer (DP8) was the main oligomer in T-ChOS. DP4 and longer oligomers was the major fraction or 93.9%.

HPLC analysis of N-acetyl glucosamine (A) and glucosamine (D) revealed that the monomer (DP1) was the major component and no oligomers were detected (results not shown).

### Bovine cartilage explant studies

### The effect of different aminosugars

### Anabolic conditions

Cartilage explants viability was not affected by the aminosugars, no significant difference was observed between various aminosugars and control groups judged by Alamar Blue assay at the end of the treatment.

Chondroitin sulphate (ChS) release in the explant media was measured from day 3 to day 23 with 2-3 days interval. There was an Oligomin dose related decrease effect for the late period (day 10-23; linear regression). When accumulated ChS release for day 10-23 was analysed (Figure 2), there was a clear Oligomin induced decrease of ChS release for the period (p<0.05 for Oligomin 200 and 400 µg/ml; 51% reduction). Glucosamine (D 400 µg/ml) had the same effect (p<0.05). T-ChOS and N-acetyl glucosamine (A) had no significant effect of the accumulated chondroitin sulphate release for the period (Figure 2).

### N-terminal propeptide of type II collagen (PIINP)

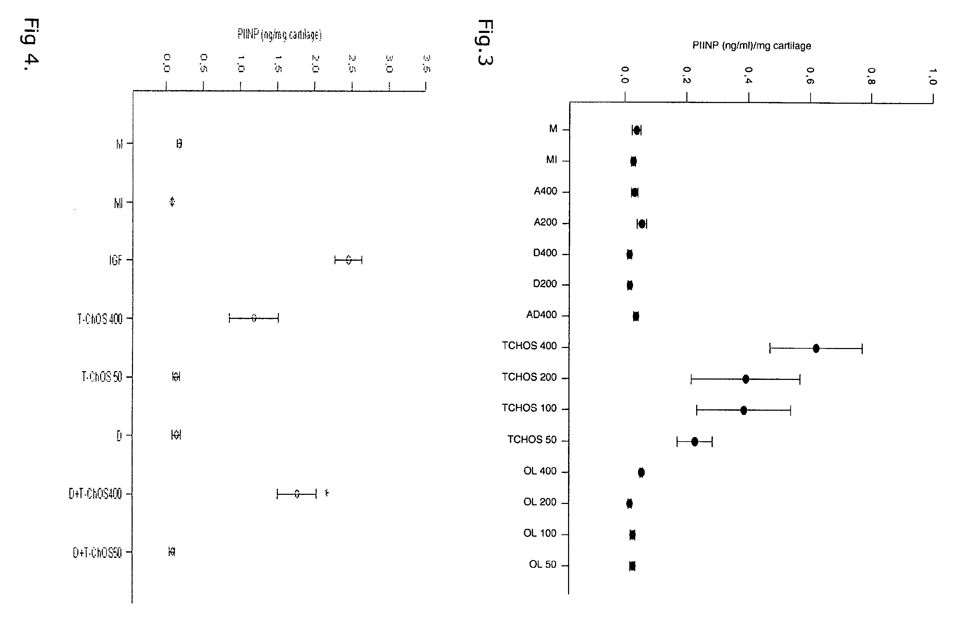

Of all biomarkers tested the collagen type II formation (PIINP concentration) was most clearly affected by the aminosugars. This was measured as released from cartilage explants into the medium over 2-3 days interval. Only T-ChOS had a strong dose related effect. This effect was only observed in the late period of aminosugar incubation. On day 20 ex vivo there was no effect observed by different aminosugars. The PIINP concentration was between 0.1 and 0.2 ng/ml mg cartilage. On day 22 there was a clear dose related increase of PIINP but only by T-ChOS (Figure 3). The effect was significantly different from zero (M) at all concentration (50, 100, 200 and 400 µg/ml) and the maximum effect was 0.62 ng/ml mg cartilage or 17-fold the control. No effect was found by Oligomin in same concentration range. The monosaccharides glucosamine (D) and N-acetyl glucosamine (A) had no effect (Figure 3). Therefore of all aminosugars tested only T-ChOS had a strong dose related effect on the PIINP expression.

For extracted cartilage protein content no significant difference was found between various aminosugars (or their concentrations) and the control. However there was a significant linear decrease of cartilage protein content with increased concentration of Oligomin (p=0.027; N=20). For Oligomin there was a linear increase of cartilage protein content with increased accumulated chondroitin sulphate (ChS) release (p=0.019; N=20).

### Combination of T-ChOS and glucosamine (T-ChOS+D)

In this set of bovine explant experiments glucosamine (200 µg/ml) was tested in combination of T-ChOS (50 and 400 µg/ml). Controls were T-ChOS only (50 and 400 µg/ml), Oligomin only (50 and 400 µg/ml) or glucosamine only. Insulin-like Growth Factor-1 (IGF-1), 100 ng/ml served as a positive anabolic stimulation control.

### N-terminal propeptide of type II collagen (PIINP)

There was a strong T-ChOS induction on the PIINP release and T-ChOS + glucosamine (400 µg/ml + 200 µg/ml) induced the PIINP further. This is apparent when looking at the accumulative effect (Figure 4). Student's t-test revealed a significant difference between T-ChOS and T-ChOS+D on the accumulated PIINP release (Figure 4). Glucosamine alone had no effect. Oligomin 50-400 µg/ml with or without 200 µg/ml glucosamine (D) did not have any effect on the PIINP release at day 20 and 24.

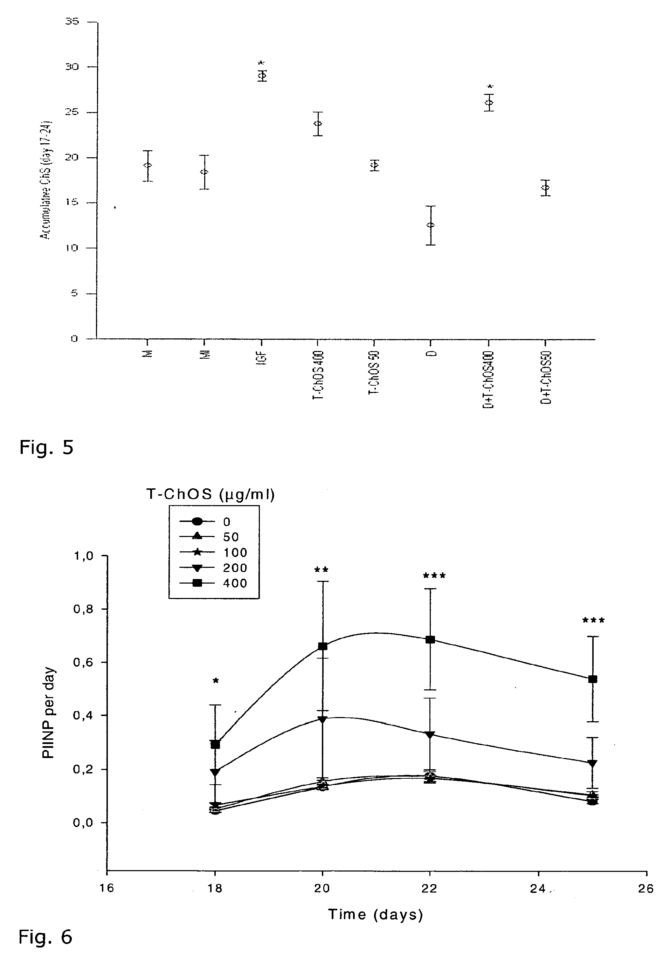

The effect of T-ChOS, T-ChOS+D and IGF-1 of the accumulative chondroitin sulphate release (day 17-24) is shown in Figure 5. Both IGF-1 and T-ChOS+D had a significant increase in the accumulative chondroidin sulphate release (day 17-24) but neither T-ChOS nor glucosamine alone did have any significant effect (Figure 5).

Extracted cartilage protein assay at day 24 did not reveal any significant effect of T-ChOS, T-ChOS+D or IGF-1.

### Human cartilage explant studies

Cartilage explants were obtained from a knee of a 40 year old women suffering from osteoarthritis. Explants were kept in growth media under anabolic conditions for 25 days. Fresh media was applied every 2-3 days. Aminosugars (Oligomin, T-ChOS, A and D; different concentration) were kept in media from day 0 to day 25.

Aggrecanase mediated aggrecan breakdown (aggrecan fragment 374ARGS-ELISA) was not affected by different aminosugars at day 9.

The collagen type II formation (PIINP concentration) was most clearly affected by the aminosugars. This was measured as released from cartilage explants into the medium over 2-3 days interval (day 18-25). Only T-ChOS had a strong dose related effect. This effect was only observed in the late period of aminosugar incubation (day 18, 20, 22 and 25; Figure 6). The maximum effect was at day 22 1.37 ng/ml mg cartilage or 3.9-fold the control. No effect was found by Oligomin in same concentration range. Glucosamine (D) had no effect. Therefore of all aminosugars tested only T-ChOS had a strong dose related effect on PIINP expression.

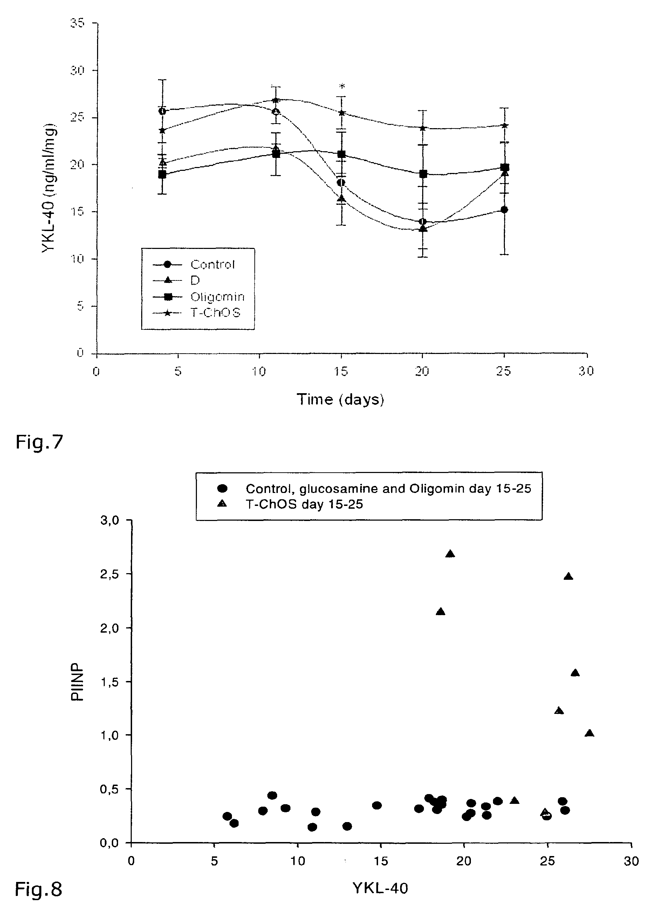

Since YKL-40 is a proposed receptor for the T-ChOS, the extracellular YKL-40 release was analyzed in media at day 4, 11, 15, 20 and 25 in explants treated with 400 µg/ml of T-ChOS, Oligomin and glucosamine (D). Figure 7 shows the effect of the different aminosugars for the period. For the control there was a significant drop in the YKL-40 expression between day 10 and 20. T-ChOS and to a lesser extent, Oligomin maintained high YKL-40 expression for the late period (day 15 -25). There was a significant difference detected on day 15 where T-ChOS maintained high YKL-40 expression. Glucosamine had no effect (Figure 7). Figure 8 shows the relationship between YKL-40 and PIINP expression for day 15-25 period for all groups tested. Only T-ChOS induced PIINP release and maintained high YKL-40 expression (a clear linear regression). This relationship disappeared when the T-ChOS group was excluded from the data (no linear regression).

Total chondroitin sulphate content (extraction of chondroitin sulphate) at day 25 was not affected by various aminosugars. Neither were total proteins levels (BioRad protein assay) nor total hydroxyproline levels in the explants at day 25.

Cell-viability of human OA articular cartilage explants at day 25 (Alamar Blue assay) were induced by the aminosugars. There was a dose related effect of all three aminosugars tested (linear regression; p<0.05). The effect was strongest by Oligomin, then by T-ChOS and the least by glucosamine.

### Example 2: The effect of T-ChOs in combination with Glucosamine on Rheumatoid Arthritis, using the Type II Collagen induced Rheumatoid Arthritis Rat Model

### Introduction

This example describes an animal study of the possible effects of chitooligosaccharides using a RA Rat Model. The ChOS mixture tested is the T-ChOS composition. The aim of this study was to investigate if a combination of glucosamine and T-ChOS would have a stronger effect than T-ChOS alone.

### Materials and Methods.

Animals used were 10 per group for arthritis-induced groups, 4/group for non-induced control group), housed 4-5/cage. For the arthritis induction, the animals were anesthetized with Isoflurane and given subcutaneous/intradermal (SC/ID) injections of 300 µl of Freund's Incomplete Adjuvant (Difco, Detroit, MI), containing 2 mg/ml bovine type II collagen (Elastin Products, Owensville, MO), at the base of the tail and 2 sites on the back on days 0 and 6. Dosing of the various oligomer and/or monomer compositions by oral route (QD at 24 hr intervals) was initiated on day 0 of the study and continued through day 16. Experimental groups were as shown in Table 1:

**Table 1**

| **Group** | **N** | **Treatment Day 0-16** |
|---|---|---|
| 1 | 4 | Normal controls+water vehicle |
| 2 | 10 | Arthritis+water vehicle |
| 3 | 10 | Arthritis+ T-ChOS (7.1 mg/kg) |
| 4 | 10 | Arthritis+ D (21.4 mg/kg) |
| 5 | 10 | Arthritis+ T-ChOS (7.1 mg/kg)+ D (21.4 mg/kg) |
| 6 | 10 | Arthritis+ T-ChOS (7.1 mg/kg)+ D (14.3 mg/kg) |
| 7 | 10 | Arthritis+ T-ChOS (7.1 mg/kg)+ D (28.6 mg/kg) |

### Animals were terminated on study day 17.

Efficacy evaluation was based on ankle caliper measurements, expressed as area under the curve (AUC), terminal hind paw weights, and histopathologic evaluation of ankles and knees. For statistical analysis various inflammation calculations were performed for the ankle diameter data, Anova and t-tests (parametrical or non-parametrical tests) were applied according to software guidelines (SigmaStat). All animals survived to study termination.

### Results

Vehicle treated disease control rats had mean body weight gain of 14 grams. Body weight gain for treatment groups did not differ significantly from disease controls.

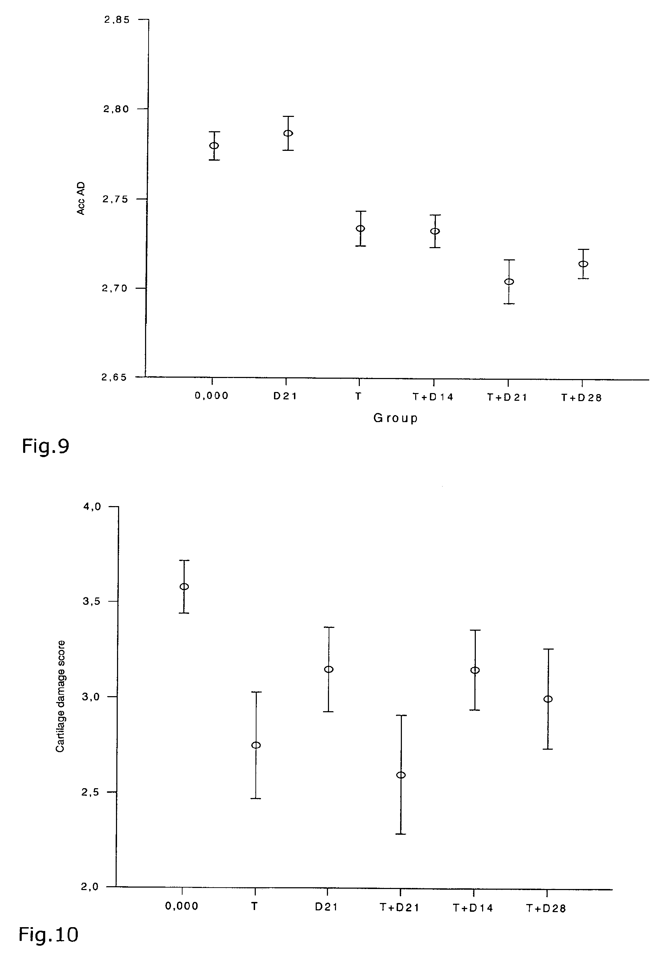

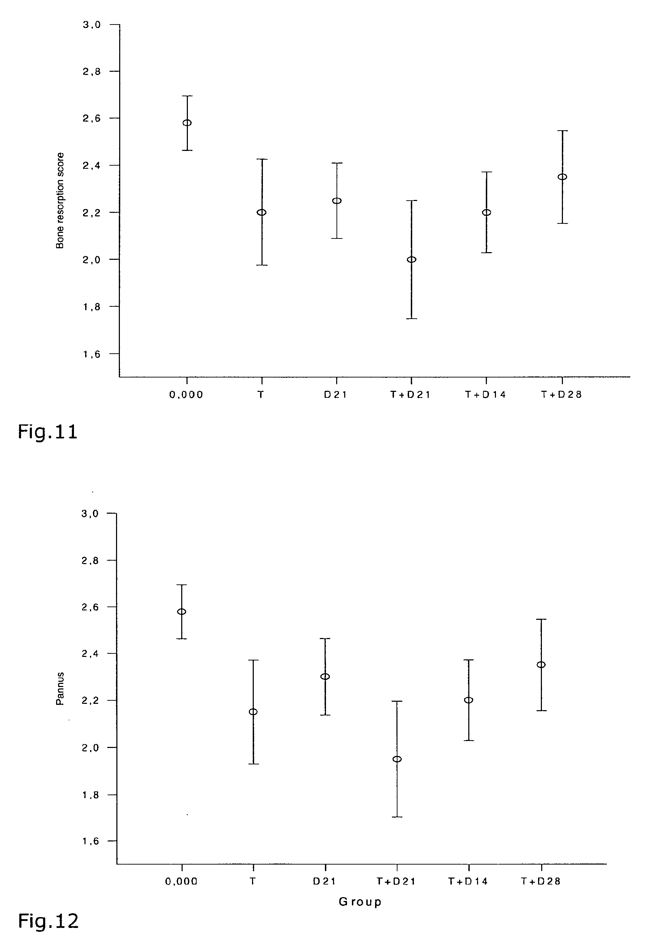

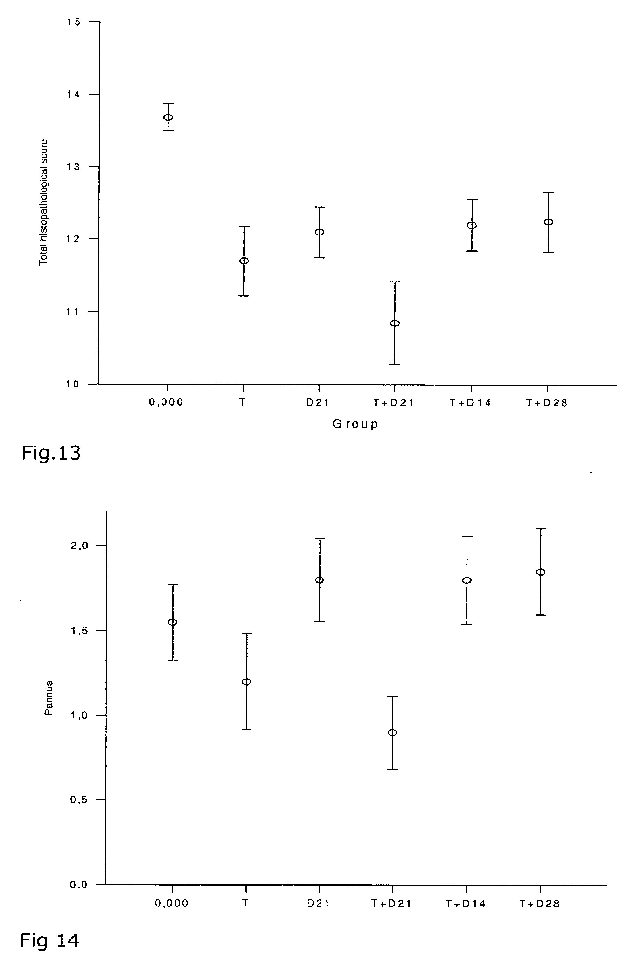

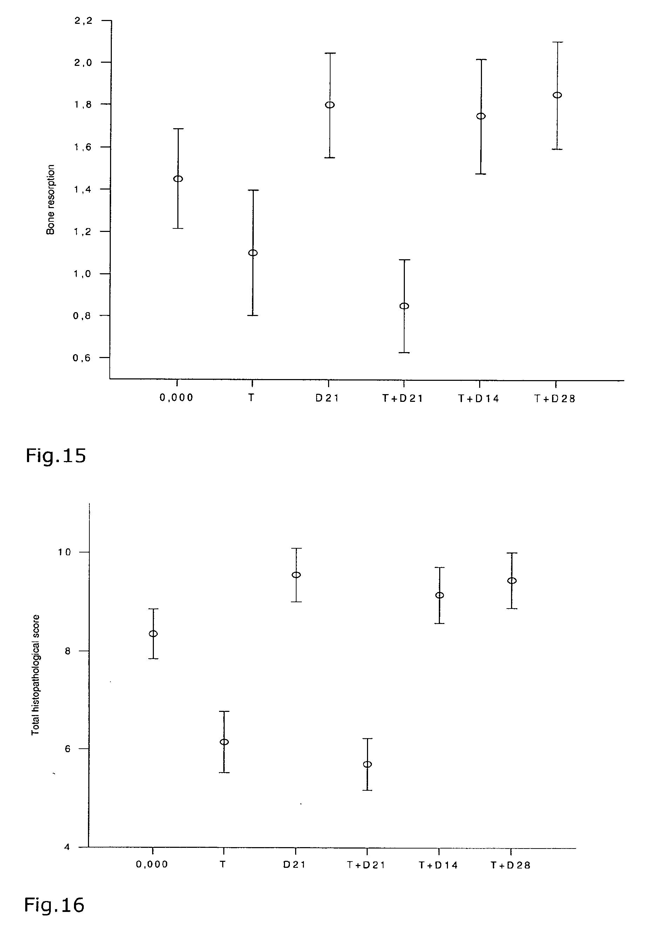

Significant reduction of ankle diameter was seen in rats treated with T-ChOS+ 21 mg/kg D (Group 5) (significant days 12-13), T-ChOS+ 14 mg D (Group 6) (d12), or T-ChOS+28 mg/kg D (Group 7) (d12, 15), as compared to disease controls. The effect of T-ChOS and glucosamine on accumulative ankle diameter (day 9-17) is shown in Figure 9. T-ChOS was effective alone as well as in all three glucosamine concentration combinations. Glucosamine alone had no effect. T-ChOS + 21 mg/kg (Group 5) had the strongest effect.
Ankle cartilage damage scores were significantly reduced towards normal in rats in Group 5 (27% reduction), as compared to disease controls (Figure 10). Only Group 5 rats showed significant reduction.
Ankle bone resorption scores were significantly reduced towards normal in rats in Group 5 (22% reduction), as compared to disease controls (Figure 11). Only Group 5 showed significant reduction.
Ankle pannus scores were significantly reduced towards normal in rats in Group 5 (24% reduction), as compared to disease controls (Figure 12). Only Group 5 showed significant reduction.
Inhibition of summed ankle histopathology parameters was significant for animals treated with all aminosugar groups (Figure 13). Group 5 rats showed the strongest effect (21%; p<0.01), then T-ChOS alone (Group 3) (14%; p<0.05), then Group 4 (12%; p<0.05) followed by Group 6 (11%) and Group 7 (10%) see Figure 13
Knee pannus scores were significantly reduced towards normal in rats treated with T-ChOS+ 21 mg/kg D (42% reduction), as compared to disease controls (Figure 14). Only T-ChOS+ 21 mg/kg D showed significant reduction.
Knee bone resorption scores were significantly reduced towards normal in rats treated with T-ChOS+D21 (41% reduction), as compared to disease controls. Only T-ChOS+21 mg/kg 21 showed significant reduction (Figure 15).
Inhibition of summed knee histopathology parameters was significant for animals treated with T-ChOS (26%; p<0.05), and significant for those treated with T-ChOS+21 mg/kg D (32%; p<0.01), as compared to disease controls (Figure 16). Summed knee scores were non-significantly increased by treatment with 21 mg/kg D (14% increase), T-ChOS+ 14 mg/kg D (10%), or T-ChOS+ 28 mg/kg D (13%), as compared to disease controls. T-ChOS+ 21 mg/kg D had the strongest effect (Figure 16).

In summary, results of this study indicated that oral, daily treatment with T-ChOS (7.1 mg/kg) + D (21.4 mg/kg) effectively inhibited ankle cartilage damage, bone resorption and pannus formation. T-ChOS (7.1 mg/kg) + D (21.4 mg/kg) also inhibited knee pannus formation and bone resorption, associated with developing type II collagen arthritis in rats. Other doses of T-ChOS in combination with D-glucosamine had sporadic significant effects on ankle caliper measures and T-ChOS alone had slight non-significant benefit. However, D-glucosamine alone had no beneficial effect.

In conclusion T-ChOS in combination with glucosamine improved the effects of T-ChOS against RA in collagen II induced rat RA model. The most effective concentration of glucosamine in the T-ChOS combination was 21 mg/kg rat.

### Example 3

### The effect of Oligomin, T-ChOS, T-ChOS+N-acetyl glucosamine and T-ChOS + glucosamine on a human rheumatic arthritis patient.

A woman diagnosed with rheumatic arthritis has been using chitooligomers produced by Genis ehf ("Oligomin") for several years in order to relieve her pain. She has rated her pain and mobility relief score in such way that 0 is no relief and 10 is complete relief. Her relief score for Oligomin (2200 mg/day) is 7.

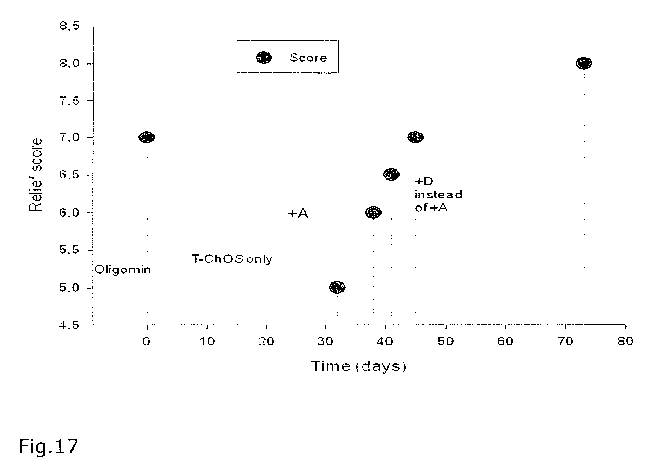

She was asked to switch to T-ChOS (700 mg/day). Gradually her conditions got worse. On day 20 her relief score was down to 5. On day 32 of T-ChOS consumption N-acetyl glucosamine (1500 mg/day) was added to the T-ChOS (Fig. 17). As a result her conditions got better. In 12 days of adding N-acetyl glucosamine to T-ChOS her relief score raised from 5 to 7 (Fig. 17). Thirteen days later her condition was unchanged (score = 7). Then she switched from N-acetyl glucosamine to glucosamine (1500 mg/day) still in combination with T-ChOS (Fig. 17). In 16 days her conditions slightly improved resulting in an increase from 7 to 8. She has now used this combination for more than a year and still her condition is the same and relief score is still 8.

### Conclusions

The examples above show thatT-ChOS is a specific combination of partially deacetylated chitooligosaccharide compositions where monomers have been removed from the combination and dimers and trimers reduced down to less than 10%, in order to increase the binding affinity to the chitinase like proteins. This is shown in Ex. 1, Aminosugar analysis (Fig. 1)

Oligomin, being a crude mixture of ChOS with large fractions of monomers, dimers and trimers, does not possess the bioactive properties shown by T-ChOS. The composition is shown in Ex. 1, Fig. 1. It is demonstrated in Ex. 1, that Oligomin does not induce collagen type II synthesis in cartilage explants while T-ChOS shows 15-20 times increase of PIINP release.

The oligosaccharide compositions have a superior tissue protective/regenerative activity over other aminosugars and chitooligosaccharide compositions. In Example 1, the PIINP release from cartilage explants is shown (Fig. 3).

The T-ChOS activity is mediated through interaction with F18 Mammalian Chitinases (Chitinase Like Proteins). This is shown in Ex. 1, T-ChOS mediates its collagen formation activity through interaction with YKL-40 (Figs. 7 and 8). Oligomin did not show any such effect (Fig. 8) in the concentration range tested.

The T-ChOS activity can be boosted in combination with the Glucosamine monomer. This is shown in Ex. 1, where PIINP release from cartilage explants is enhanced by T-ChOS. The combination of ChOS and Glucosamine is also significantly more efficient than T-ChOS alone (Fig. 4). In Ex. 1, the Chondroitin Sulfate (ChS) release from cartilage explants, effect of T-ChOS + Glucosamine is close to the effect of insulin-like growth factor-I (IGF-I) (Fig. 5). Furthermore in Ex. 2, Figs. 19-26, oral administration of T-ChOS and glucosamine together in a Rheumatoid Arthritis rat model is superior over T-ChOS or glucosamine alone.

T-ChOS and glucosamine will exert their activity *in vivo* after oral administration. This is shown in Ex 3, Figs. 4-8, where oral administration of T-ChOS and glucosamine together has a significant tissue protective effect in Rheumatoid Arthritis model in rats.

## Claims

1. A composition for treatment of rheumatic arthritis, comprising:
- therapeutically active chitooligomers of N-acetyl glucosamine (A) and glucosamine (D), wherein the chitooligomers comprise hetero-chitooligomers which fulfill the following criteria:
- said oligomers having a chain length in the range of 5-20 monomer residues
- each oligomer chain can have two N-acetyl glucosamine residues (AA) on either or both ends of the oligomer chain,
- the remaining internal part of the oligomer having at least enough amount of D residues to avoid that the sequence of said internal chain comprises an N-acetyl glucosamine residue (A) adjacent to another N-acetyl glucosamine residue (such as AA),
- in combination with glucosamine,
- wherein the ratio between monomers and oligomers is in the range between about 1:5 to about 5:1, and
- wherein the degree of deacetylation (DD) of said therapeutically active chitooligomers is in the range between 30 - 60%.

2. The composition according to claim 1, wherein said ratio between monomers and oligomers is in the range between about 1:2 to about 4:1.

3. The composition according to any of the preceding claims, wherein the biomaterial further comprises a component selected from any of the following: calcium phosphates, including hydroxyapatite, calcium sulphate, sodium tripolyphosphate, alginate, collagen hyaluronic acid and chitosan polymer.

4. The composition of any one of claims 1 to 3 formulated in a form selected from the group consisting of a powder, a suspension, a gel, a sol, aerosol, a paste, a film, a foam, a pill, and a capsule.

5. The composition of claim 4 comprising a pharmaceutically acceptable excipient.

## Patentansprüche

1. Zusammensetzung zur Behandlung von rheumatoider Arthritis, umfassend:
- therapeutisch wirksame Chitooligomere von N-Acetylglucosamin (A) und Glucosamin (D), wobei die Chitooligomere Heterochitooligomere umfassen, die die folgenden Kriterien erfüllen:
- die Oligomere haben eine Kettenlänge im Bereich von 5-20 Monomerresten
- jede Oligomerkette kann an einem beliebigen oder beiden Enden der Oligomerkette zwei N-Acetylglucosaminreste (AA) aufweisen,
- der restliche innere Teil des Oligomers hat mindestens eine genügende Menge an D-Resten, um zu vermeiden, dass die Sequenz der inneren Kette einen N-Acetylglucosaminrest (A) umfasst, der mit einem anderen N-Acetylglucosaminrest benachbart ist (wie AA),
- in Kombination mit Glucosamin,
- wobei das Verhältnis zwischen Monomeren und Oligomeren im Bereich zwischen etwa 1:5 bis etwa 5:1 liegt und
- wobei der Deacetylisierungsgrad (DD) der therapeutisch wirksamen Chitooligomere im Bereich zwischen 30-60% liegt.

2. Zusammensetzung nach Anspruch 1, wobei das Verhältnis zwischen Monomeren und Oligomeren im Bereich zwischen etwa 1:2 und etwa 4:1 liegt.

3. Zusammensetzung nach einem der vorhergehenden Ansprüche, wobei das Biomaterial weiterhin eine aus den Folgenden ausgewählte Komponente umfasst:
Calciumphosphate einschließlich Hydroxyapatit, Calciumsulfat, Natriumtripolyphosphat, Alginat, Kollagen, Hyaluronsäure und Chitosanpolymer.

4. Zusammensetzung nach einem der Ansprüche 1 bis 3, formuliert in einer Form ausgewählt aus der Gruppe bestehend aus einem Pulver, einer Suspension, einem Gel, einem Sol, einem Aerosol, einer Paste, einem Film, einem Schaum, einer Pille und einer Kapsel.

5. Zusammensetzung nach Anspruch 4, umfassend einen pharmazeutisch unbedenklichen Exzipienten.

## Revendications

1. Composition destinée au traitement de la polyarthrite rhumatoïde, comprenant :
- des chito-oligomères thérapeutiquement actifs de N-acétylglucosamine (A) et de glucosamine (D), où les chito-oligomères comprennent des hétéro-chito-oligomères répondant aux critères suivantes :
- lesdits oligomères présentent une longueur de chaîne comprise entre 5 et 20 résidus monomères
- chaque chaîne oligomère peut porter deux résidus N-acétylglucosamine (AA) sur chacune ou les deux extrémités de la chaîne oligomère,
- la partie interne restante de l'oligomère ayant au moins une quantité suffisante de résidus D pour éviter que la séquence de ladite chaîne interne ne comprenne un résidu N-acétylglucosamine (A) adjacente à un autre résidu N-acétylglucosamine (tel que AA),
- en combinaison avec de la glucosamine,
- où le rapport entre les monomères et les oligomères est compris dans l'intervalle entre environ 1:5 et environ 5:1, et
- où le degré de désacétylation (DD) desdits chito-oligomères thérapeutiquement actifs est compris dans l'intervalle entre 30 et 60 %.

2. Composition selon la revendication 1, où ledit rapport entre les monomères et les oligomères est compris dans l'intervalle entre environ 1:2 et environ 4:1.

3. Composition selon l'une quelconque des revendications précédentes, où le biomatériau comprend en outre un composant choisi parmi l'un quelconque des suivants : phosphates de calcium, y compris hydroxyapatite, sulfate de calcium, tripolyphosphate de sodium, alginate, collagène, acide hyaluronique et polymère de chitosane.

4. Composition selon l'une quelconque des revendications 1 à 3 formulée dans une forme choisie dans le groupe constitué par une poudre, une suspension, un gel, un sol, un aérosol, une pâte, une pellicule, une mouche, une pilule et une capsule.

5. Composition selon la revendication 4 comprenant un excipient pharmaceutiquement acceptable.
